(19) Europäisches Patentamt
European Patent Office
Office européen des brevets

(11) Publication number: **0 038 576**
**B1**

(12) # EUROPEAN PATENT SPECIFICATION

(45) Date of publication of patent specification: **11.04.84**

(51) Int. Cl.³: **C 07 D 307/94,**
A 61 K 31/34 //C07D307/32

(21) Application number: **81104398.3**

(22) Date of filing: **01.05.80**

(60) Publication number of the earlier application in accordance with Art. 76 EPC: **0018830**

(54) **7-acetylspirobenzofuranone compound.**

(30) Priority: **04.05.79 JP 55082/79**
**25.06.79 JP 80551/79**

(43) Date of publication of application:
**28.10.81 Bulletin 81/43**

(45) Publication of the grant of the patent:
**11.04.84 Bulletin 84/15**

(84) Designated Contracting States:
**BE CH DE FR GB IT LI NL SE**

(56) References cited:
**EP - A - 0 003 084**

(73) Proprietor: **Takeda Chemical Industries, Ltd.**
**27, Doshomachi 2-chome Higashi-ku**
**Osaka-shi Osaka, 541 (JP)**

(72) Inventor: **Sugihara, Hirosada**
**5-604, 2 Minase 2-chome Shimamotocho**
**Mishima-gun Osaka 618 (JP)**
Inventor: **Watanabe, Masazumi**
**4-54, Seiwadainishi 2-chome**
**Kawanishi Hyogo 666-01 (JP)**
Inventor: **Kawada, Mitsuru**
**25-3, Tsukaguchicho 4-chome**
**Amagasaki Hyogo, 661 (JP)**
Inventor: **Imada, Isuke**
**18-10, Tsuruyamadai 4-chome**
**Izumi Osaka 594 (JP)**

(74) Representative: **Lewin, John Harvey et al,**
**Elkington and Fife High Holborn House**
**52/54 High Holborn**
**London WC1V 6SH (GB)**

**EP 0 038 576 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

7-acetylspirobenzofuranone compound

This invention relates to a 7-acetylspirobenzofuranone compound, a process for its preparation, and the use of said compound.

The present compound is 7-acetylspiro[benzo[b]-furan-2(3H), 1′-cyclopropane]-3-one having the formula (I):

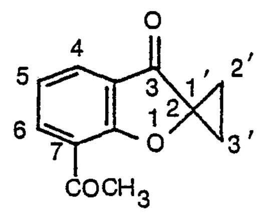

(I)

The compound (I) of the present invention can be produced, for example, by subjecting a compound of the formula (II):

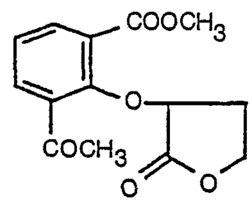

(II)

to condensation and decarboxylation in one step with an organic base and a catalyst which assists decarboxylation.

In the above reaction, the organic base is employed to promote ester-condensation, i.e. Dieckmann condensation, and the decarboxylating catalyst is employed to promote decarboxylation of the condensed compound [the formula (III) described hereinafter]. Preferred examples of said organic base include tertiary amines (e.g. triethylamine, 1,4-diazabicyclo[2,2,2]octane or 1,8-diazabicyclo[5,4,0]-7-undecene). Preferred examples of said catalyst which assists decarboxylation include metal halides (e.g. sodium chloride, sodium bromide, sodium iodide, potassium bromide, potassium chloride and potassium iodide) and quaternary ammonium salts (e.g. tetramethylammonium bromide). The reaction temperature is normally about 100°C to 200°C and preferably about 140°C to 160°C, although the reaction may be conducted at higher or lower temperatures if it is desired to control the reaction velocity. Purging the reaction vessel with an inert gas (e.g. $N_2$, argon) is sometimes effective in preventing side reactions and improving yields. This reaction is normally carried out in a suitable solvent. While any solvent that will not interfere with the reaction may be employed, it is normally advantageous to employ a solvent having a boiling point higher than the reaction temperature (e.g. dimethyl sulphoxide, N,N-dimethylformamide, hexamethylphosphoramide).

The compound (I) of the present invention can be produced also by subjecting a compound of the formula (III):

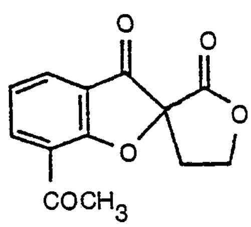

(III)

to decarboxylation.

This reaction is carried out in the presence of the above-mentioned decarboxylating catalyst and under similar reaction conditions to those described in the condensation and decarboxylation reaction of the compound (II), but in the absence of the organic base which promotes ester condensation.

The contemplated compound (I) obtained in the foregoing manner can be isolated from the reaction mixture and purified by conventional procedures (e.g. distillation, recrystallization or column chromatography).

The spiro compound (I) according to this invention is a new compound which exhibits gastric secretion inhibitive, antiinflammatory, analgesic and other effects in mammels (e.g. man, rat, mouse, guinea-pig, dog and pig). Therefore, it is of value as an antiulcer, antiinflammatory or analgesic agent,

and as drugs for the management of peptic ulcer, acute or chronic gastritis, lumbago, arthritis and other diseases. Management of a peptic ulcer in accordance with the present invention includes both the prophylactic administration of the spiro compound (I) to prevent the outbreak of an ulcer in an ulcer prone patient, as well as the treatment of an existing peptic ulcer. In such medicinal application, the compound (I) can be safely administered orally or parenterally, either as it is or as formulated with pharmaceutically acceptable carriers or diluents known per se into suitable dosage forms such as tablets, powders, capsules, injections and suppositories. While the recommended dosage depends on the subject, condition, route of administration, etc., the normal oral dosage for the treatment of peptic ulcer or acute or chronic gastritis is about 1 mg. to 20 mg. as compound (I) per kg body weight per dose, to be given once to 3 times daily.

The starting compounds (II) and (III) which are employed in the practice of this invention can be prepared by the following route of synthesis or any process analogous thereto.

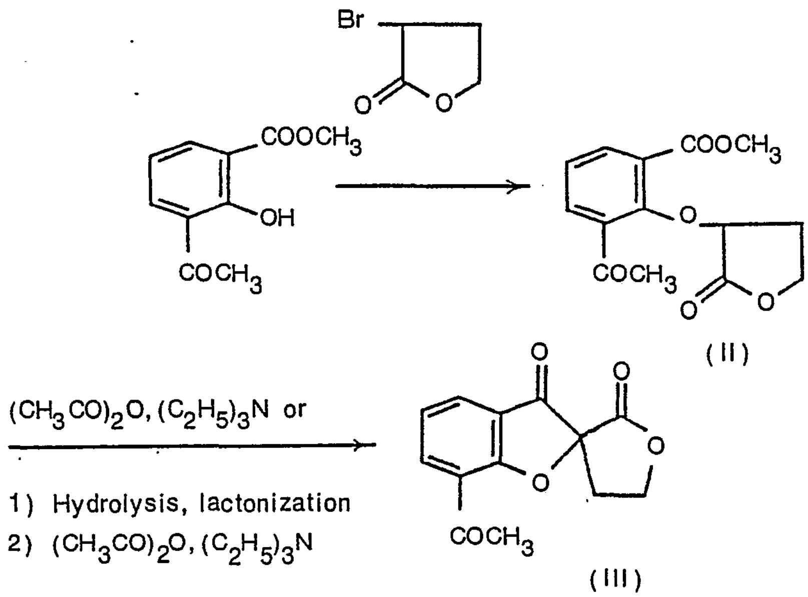


(II)

(III)

The following reference and working examples are intended to describe this invention in further detail.

Reference Example

α-Bromo-γ-butyrolactone (12.5 g) was added to a mixture of methyl 3-acetylsalicylate (5.8 g) and potassium carbonate (10.4 g) in acetone (200 ml) under stirring, and the mixture was refluxed for 11 hours. The resulting mixture was worked up in a similar manner to the corresponding step described in Reference Example 4 of EP—A—0018830. By this procedure, there was obtained α-[(6-acetyl-2-methoxycarbonylphenyl)oxy]-γ-butyrolactone as an oil.

Elemental analysis, for $C_{14}H_{14}O_6$

Calcd.: C, 60.43; H, 5.07

Found: C, 60.21; H, 5.02

Example

A mixture of α-[(6-acetyl-2-methoxycarbonylphenyl)oxy]-γ-butyrolactone (3.5 g), 1,8-diazabicyclo[5,4,0]-7-undecene (0.14 g) and sodium chloride (1.1 g) in N,N-dimethylformamide (66.5 ml) was stirred at 150—160°C for 5 hours. The solvent was distilled off under reduced pressure and the residue was subjected to chromatography on silica gel. The fraction eluted with dichloromethane was recrystallized from $CHCl_3$-hexane to give 7-acetyl-spiro[benzo[b]furan-2(3H), 1′-cyclopropane]-3-one (0.22 g) as colorless neddles melting at 114—115°C.

Elemental analysis, for $C_{12}H_{10}O_3$

Calcd.: C, 71.28; H, 4.99

Found: C, 71.39; H, 4.96

## Claims

1. 7-Acetylspiro[benzo[b]furan-2(3H), 1′-cyclopropane]-3-one of the formula:

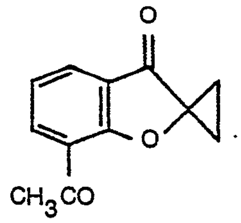

2. A pharmaceutical composition which comprises, as an active ingredient, an effective amount of the compound as claimed in Claim 1, and a pharmaceutically acceptable carrier or diluent therefor.

3. The compound as claimed in Claim 1 or a pharmaceutical composition as claimed in Claim 2 for use in therapeutical treatment of a mammal.

4. A process for producing the compound as claimed in Claim 1, characterized by subjecting $\alpha$-[(6-acetyl-2-alkoxy-carbonylphenyl)oxy]-$\gamma$-butyrolactone of the formula:

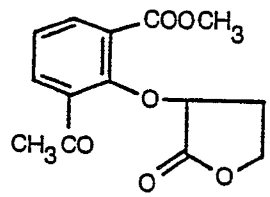

to Dieckmann condensation in the presence of an organic base and a catalyst which assists decarboxylation, or by decarboxylating 7-acetyl-4′,5′-dihydrospiro[benzo[b]furan-2(3H), 3′(2′H)-furan]-2′,3-dione of the formula:

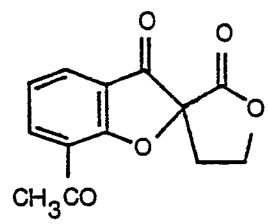

in the presence of a catalyst which assists decarboxylation.

## Revendications

1. 7-acétylspiro[benzo[b] furanne-2(3H), 1′-cyclopropane]-3-one ayant la formule:

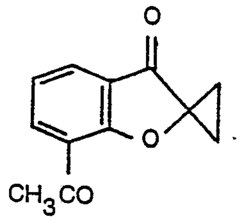

2. Composition pharmaceutique qui comprend, comme ingrédient actif, une quantité efficace du composé revendiqué dans la revendication 1, et un véhicule ou un diluant pour ce composé pharmaceutiquement acceptable.

3. Composé revendiqué dans la revendication 1, ou composition pharmaceutique revendiquée dans la revendication 2, utilisables dans le traitement thérapeutique des mammifères.

4. Procédé pour la préparation du composé revendiqué dans la revendication 1, caractérisé par le fait qu'on soumet 1′$\alpha$-[(6-acétyl-2-alcoxycarbonylphényl)oxy]-$\gamma$-butyrolactone ayant la formule:

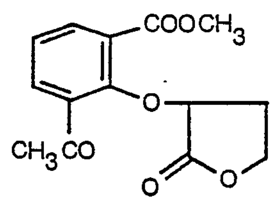

à une condensation de Dieckmann, en présence d'une base organique et d'un catalyseur qui facilite la décarboxylation, ou en décarboxylant la 7-acétyl-4',5'-dihydrospiro[benzo[b]furanne-2(3H), 3'(2'H)-furanne]-2',3-dione ayant la formule:

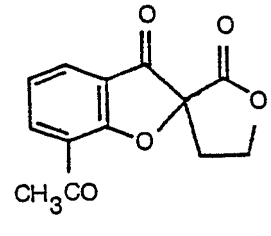

en présence d'un catalyseur qui facilite la décarboxylation.

**Patentansprüche**

1. 7-Acetylspiro[benzo[b]furan-2(3H),1'cyclopropan]-3-on der Formel

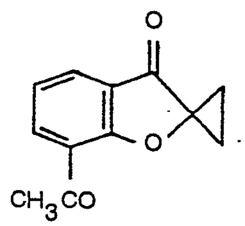

2. Pharmazeutische Zusammensetzung, enthaltend als Wirkstoff eine wirksame Menge der Verbindung nach Anspruch 1 und ein pharmazeutisch unbedenkliches Trägermaterial oder Verdünnungsmittel für diese.

3. Verbindung nach Anspruch 1 oder pharmazeutische Zusammensetzung nach Anspruch 2 zur Verwendung bei der therapeutischen Behandlung von Säugern.

4. Verfahren zur Herstellung der Verbindung nach Anspruch 1, dadurch gekennzeichnet, daß $\alpha$-[(6-Acetyl-2-alkoxy-carbonylphenyl)oxy]-$\gamma$-butyrolacton der Formel

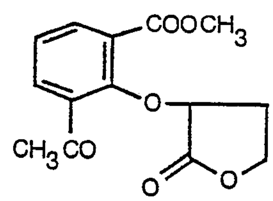

einer Dieckmann-Kondensation in Gegenwart einer organischen Base und eines Katalysators, der die Decarboxylierung unterstützt, unterworfen wird oder daß 7-Acetyl-4',5'-dihydrospiro[benzo[b]furan-2(3H),3'-(2'H)-furan]-2',3-dion der Formel

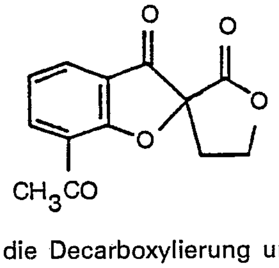

in Gegenwart eines Katalysators, der die Decarboxylierung unterstützt, decarboxyliert wird.